# EUROPEAN PATENT APPLICATION

(11) **EP 3 216 394 A1**
(43) Date of publication of application: **13.09.2017**
(21) Application number: 16159585.5
(22) Date of filing: 10.03.2016
(51) Int. Cl.: A61B 5/145, A61B 5/00, A61B 5/1486

(54) **MEDICAL SYSTEM FOR MONITORING AN ANALYTE IN BODY FLUID**

(71) Applicant: Roche Diabetes Care GmbH, 68305 Mannheim (DE); F. Hoffmann-La Roche AG, 4070 Basel (CH)
(72) Inventor: KUBE, Oliver, 67549 Worms (DE)
(74) Representative: Pfiz, Thomas

(57) **Abstract**

The invention concerns a medical system for monitoring an analyte in a body fluid comprising a sensor unit (12) including a subcutaneously implantable sensor (24), an electronics unit (14) configured for receiving a measurement signal from the sensor unit (12), and a power supply unit (50) for at least powering the electronics unit (14). For long-term wearability and flexible differentiation, it is proposed that the sensor unit (12) and the electronics unit (14) are arranged as physically independent entities, wherein the sensor unit (12) is construed without rigid mounting components and a connection means (18) is provided between the sensor unit (12) and the electronics unit (14) at least for transferring measurement signals.

## Description

The invention relates to a medical system for continuous monitoring an analyte in a body fluid comprising a sensor unit including a pad attachable to a body of a user and a sensor which has a subcutaneously implantable electrode portion and a contact portion arranged on the pad, and further comprising an electronics unit configured for receiving a measurement signal from the sensor unit and a power supply unit for at least powering the electronics unit.

Monitoring of certain analytes or agents, specifically glucose or lactate in body fluids like blood or interstitial fluid may be ordinarily performed by so called spot-monitoring on samples extracted from the body or by continuous monitoring via a fully or partially implanted sensor, specifically an electrochemical sensor.

In the latter case, to which the invention relates, the subcutaneously implanted sensor remains in the interstitial tissue over an extended period of time even up to several weeks. Then, the *in vivo* detected measurement signals may be indicative of an analyte, e.g. glucose in the blood of the subject. The monitoring may be a nearly real-time continuous or quasi continuous or periodic approach for frequently providing/updating analyte values without sample handling or similar user interaction.

In present practice, continuous glucose monitoring (CGM)-systems include a so-called bodymount as a rigid mounting base or platform on a flexible plaster on which the electronics unit is mounted physically associated with the sensor. In this context, it is known to from US2011/00060196 to achieve modularity by having a mounting unit with a contoured patch form of a nonwoven polymeric material as a seat and a retention mechanism disposed in the seat. The retention mechanism is insert molded directly into or on the mounting unit such that a single unitary structure is formed, where the retention mechanism is configured to releasably engage a medical device. However, such an arrangement lays off the body in considerable dimensions and does not provide additional wearing comfort.

On this basis the object of the invention is to further improve the known systems and to provide a design which can be adapted to different applications and allows flexible and easy to handle use as well as long-term wear capability.

The combination of features stated in the independent claims is proposed to achieve this object. Advantageous embodiments and further developments of the invention are derived from the dependent claims.

The invention is based on the idea of arranging the electronics unit physically independent from the sensor unit. Accordingly, it is proposed that the sensor unit and the electronics unit are arranged as physically separate entities which in use are disposed at a distance from each other at different sites on or near the body, wherein the sensor unit is construed as a flexible assembly without rigid mounting base or platform on the pad and a connection means is provided between the sensor unit and the electronics unit at least for transferring the measurement signal. Thus, the electronics unit is not mounted physically associated with the sensor, and hence the system avoids a bodymount or other platform or rigid connector componentry on the flexible plaster pad. Avoiding rigid assembly parts above the sensor reduces shear stresses during skin movements and reduces the risk of detachment. Moreover, a very compact and flat sensor tag can be realized. Such a system concept allows high use flexibility and long-term wear ability due to the decoupled arrangement of signal sensing and processing.

Advantageously, the connection means is formed as a flexible connection cable, such that a distance between sensor and electronics units can be bridged.

It is also conceivable that the energy for the sensor and/or the measurement signal readings may be provided via a wireless connection means. This may be accomplished via inductive coupling or other near field energy transmission techniques, and a buffer capacitor may be built into the sensor unit to store energy and to balance potential transmission drops. For providig the measurement signals to the electronics unit, an RFID may be arranged on the sensor unit.

Preferably, the connection cable has a first plug-free end which is non-detachably joined to the contact portion of the sensor to permanently maintain an electrical connection while keeping sufficient flexibility. Alternatively, the connection means or cable may be provided as a prolonged extension of the sensor without joints on the side of the sensor unit.

Another improvement provides simplified handling when a second end of the connection cable is provided with a plug connector preferably formed as a flat quick-connect termination, and when the electronics unit has a plug socket to receive the plug connector.

In this connection it is further advantageous when the connection cable has a length in the range of 3 to 30 cm, preferably 5 to 10 cm. The cable should be shielded against electromagnetic interference. It is also conceivable to use a cable extension e.g. in hospital use when wireless transmitters are not allowed.

In an alternative compact configuration, the connection means may be formed without a cable by a board-to-board connector including electric lines integrated to a plaster patch which carries at least one of the sensor unit, the electronics unit and the power supply unit.

In an advantageous embodiment, the sensor unit is formed as a flat layered assemblage which has a height of less than 3 mm, preferably less than 1 mm. It is also preferred that the sensor unit has a lateral dimension of less than 5 cm, preferably less than 2 cm and most preferably less than 1 cm. Such a minimally designed sensor tag allows reducing the skin surface covered by an adhesive and hence reduces the risk of skin irritations. Additionally, the risk of the sensor tag falling off due to degradation of the adhesive is reduced, because it has to carry less weight.

A particular embodiment further comprises a reusable or disposable inserter for placing the sensor unit on the skin while inserting the subcutaneously implantable electrode portion of the sensor into subcutaneous tissue. The inserter can have a very simple mechanism for insertion based on e.g. a spring drive.

The system may comprise the power supply as a third unit which is arranged separately from the sensor unit and from the electronics unit and is provided with a cable-based or wireless energy transfer means. This is particularly advantageous in the context of redundant sensor arrangements using more than one sensor unit for prolonged wear times. Redundant sensors may also be used for comparing signals in order to detect system fails. In case of a time-overlapping or staggered use of at least two sensor units, the unit applied at a later time may be calibrated by means of the earlier used unit, and a continuous measurement without disruption is feasible.

In order to achieve additional values, the electronics unit may comprise a transmitter for preferably wireless transmission of signals or analyte data to a data receiving unit, which can be a non-bodyworn or handheld device such as a smartphone, smartwatch or insulin delivery device. The data processing for deriving the analyte data from the measurements signals may be done on the electronics unit or on the receiving unit. In the first case, data ready for display can be send to multiple devices without having to make sure that the correct data processing including smoothing, filtering and calibration of the measurement current signals is installed.

According to a preferred implementation, the system further comprises a receiver configured to receive analyte data from the electronics unit and including a monitoring software to process and/or display the analyte data.

The data transmission between electronics unit and receiver may be provided in a continuous or quasi-continuous mode.

Alternatively, the data transmission between electronics unit and receiver may be provided in a discontinuous mode on demand of the user. This may be accomplished via an RFID tag in the electronics unit which transfers data whenever the user brings the receiver in close proximity to the electronics unit.

In this connection, it is also advantageous when the receiver is provided as a customized remote control device to co-operate with the electronics unit or as a smartphone including control software in the form of a mobile app.

Preferably, the electronics unit and/or the power unit is attachable to the body via a hook-and-loop plaster connection. This allows to releasably adhere to various sites during the wear time and to reduce skin irritations. Furthermore, different units can easily be changed, depending on the situation of the user.

In another advantageous embodiment, the electronics unit may be a fully independent wearable which can be carried by the user for example in a pocket or in the form of a watch.

In the following, the invention is further elucidated on the basis of embodiment examples shown schematically in the drawings, where
- Fig. 1: is a perspective view of a medical system comprising a body-mountable discrete sensor and electronics units and a receiver;
- Fig. 2: is a perspective view of the sensor unit comprising a cable connector and the electronics unit prior to connection;
- Fig. 3: is a cross-sectional view of the sensor unit with skin-inserted sensor and connector cable;
- Fig. 4: is a perspective view of the electronics unit comprising a hook-and-loop pad;
- Fig. 5: is a top view illustrating variable mounting of the electronics unit on the body connected to a fixed sensor unit;
- Fig. 6: is a top view of a further embodiment of the medical system comprising discrete sensor, electronics and power supply units;
- Fig. 7: is a top of a further embodiment of the medical system comprising a board-to-board connector between sensor and electronics unit; and
- Fig. 8: is a sectional view of an inserter for mounting the electronics unit on the body of a user.

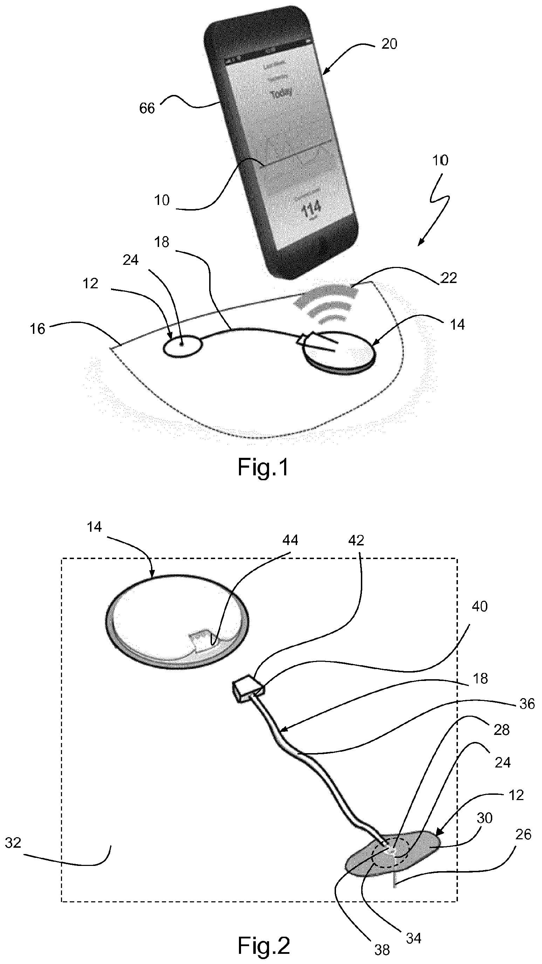

In Fig. 1, an exemplary embodiment of a medical system for monitoring an analyte in a body fluid, specifically glucose in interstitial fluid is shown. The system 10 at least comprises a sensor unit 12 and an electronics unit 14 which are arranged physically independent from each other and which in use on the body 16 of a patient are connected by a connection means 18 at least for transferring measurement signal measured by the sensor unit 12 to the electronics unit 14. The system 10 may further comprise a receiver 20 configured to receive analyte data from the electronics unit 14 e.g. via a wireless connection 22.

As apparent from Fig. 2, the sensor unit 12 includes an electro-chemical analyte sensor 24 which has a distal electrode portion 26 inserted into subcutaneous tissue and a proximal contact portion 28 arranged on a pad 30. The pad 30 is configured for adhesive attachment on the skin 32 of the body part 16, while the sensor 24 penetrates the pad 30 and is covered by a protective sheet 34 as illustrated in phantom lines. In such a compact assembly, the sensor unit 12 may have a running life in the implanted sensor state of e.g. one, two or up to four weeks of attachment to the skin.

As known *per se* to the skilled person the electro-chemical sensor 24 may comprise a flexible substrate as a carrier for a plurality of distal electrodes and proximal contact pads which are interconnected by electrical traces. The exposed electrodes are adapted for performing an electrochemical detection reaction for detecting an analyte (e.g. glucose) in body fluid (e.g. interstitial fluid or blood). For this purpose, a test chemical may be deposited on a working electrode and a counter electrode may be adapted for balancing a current flow required by the detection reaction at the working electrode. Additionally, a reference electrode may provide a stable electrode potential. A current as low as a few nanoamperes may result as a measurement signal, which has to be conducted to the electronics unit 14 for signal processing.

For this purpose, the connection means 18 is provided as a flexible connection cable 36 to bridge the distance between the fixedly inserted sensor unit 12 and the electronics unit 14 which can be positioned freely in the reach of the cable 36. In order to establish a flat and secure galvanic connection, a first end 38 of the cable 36 is non-detachably joined to the contact portion 28 of the sensor 24. The permanent electrical connection is maintained by bonding individual conductors included in the cable 36 to associated contact pads on the contact portion 28, e.g. by conductive adhesive bonding or soldering. Then, the voltage applied to the sensor 24 and the detected signals can be transferred in through a steady electroconductive joint. The cable 36 may be shielded e.g. in the form of a round co-axial cable to suppress interference signals. It is also conceivable to avoid a joint contact portion by a prolonged proximal section of the sensor 24 forming the connection means.

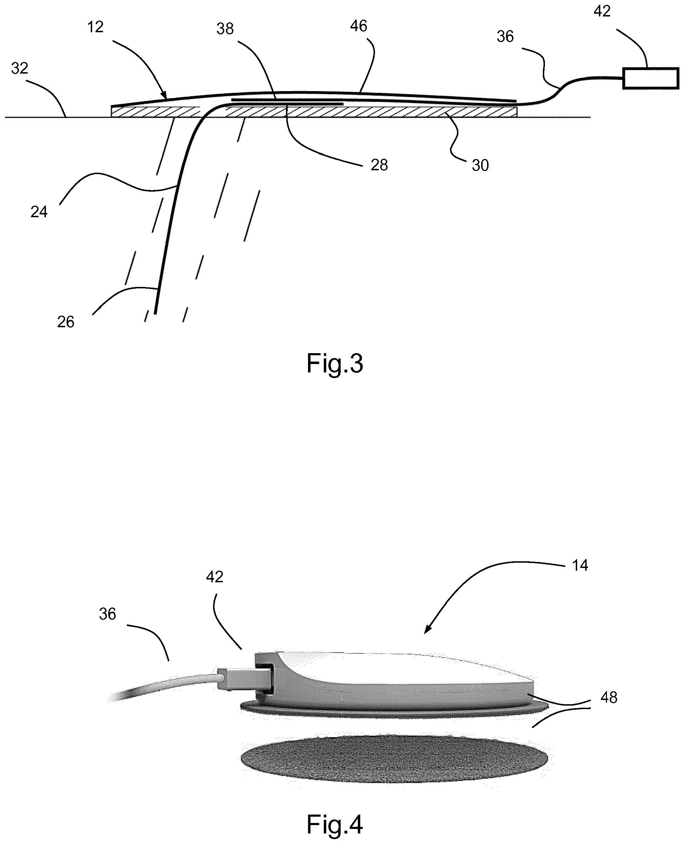

The free second end 40 of the cable 36 (or the proximally prolonged sensor) is provided with a plug connector 42 which mates with a plug socket 44 on the electronics unit 14. Thus, a user may establish the electrical connection before attaching the electronics unit 14 to the skin 32. Accordingly, the electronics unit 14 may be disconnected for replacement or repositioning. For sufficient ease of use, the cable 36 may have a length in the range of 5 to 10 cm. As apparent from Fig. 3, with the sensor unit 12 comprising only the sensor 24 and the plug-free electrical connection on the flexible pad 30, but no rigid components such as a bodymount or platform on which the electronics unit 14 would be mounted physically associated with the sensor, a very compact and flat sensor tag can be realized. The sensor 24 and the cable 36 may be fitted to thin substrates and combined with the plaster pad 30 and a cover foil 46 to achieve a flat layered assemblage which has a height of preferably less than 1 mm and lateral dimensions in the covered area of less than 2 or even 1 cm.

As depicted in Fig. 4, the self-contained electronics unit 14 may be attached to the body via a hook-and-loop (pile) plaster connection 48. This allows to easily rearrange or change the electronics unit 14, for example in case of decreasing battery power.

The electronics unit 14 comprises an amplifier for amplification of the analogue measurement signal received from the sensor 24 and a transmitter for transmitting analyte data derived from the measurement signals to a preferably outside receiver. Further components of the electronics unit 14 may include a battery or accumulator and a memory for intermediate data storage. The interior of the electronics unit 14 is not further detailed in the drawings. In case of a replacement of the electronics unit 14 during the wear time of the sensor unit 12, calibration data for the sensor may be transferred to the subsequent electronics unit 14 e.g. by a wireless transfer via the receiver 20.

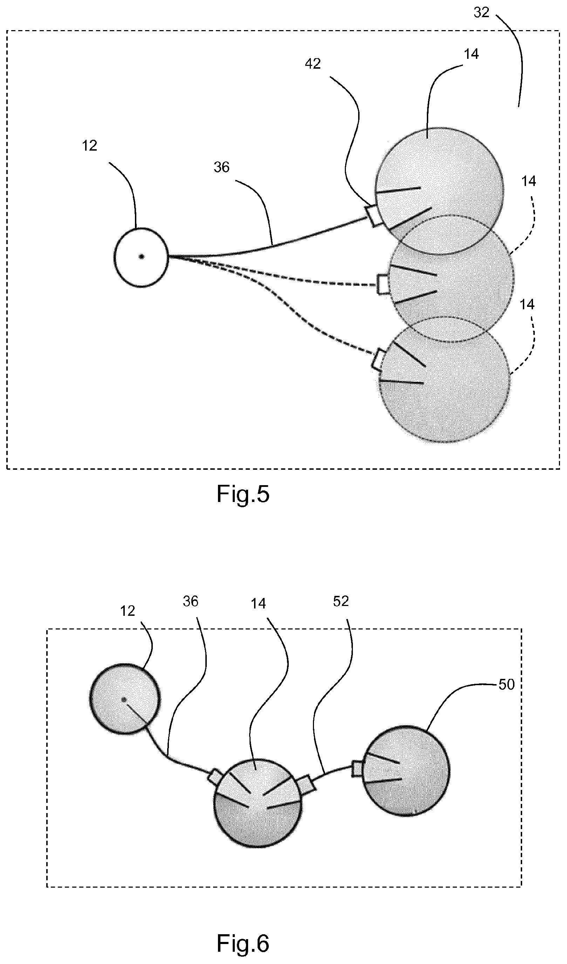

Fig. 5 illustrates that the electronics unit 14 may be adhered to various sites on the skin during the wear time of the fixedly implanted sensor unit 12. Such a relocation allows to reduce skin irritations and to account for eventually reduced adherence of the larger and heavier electronics unit 14. It is also possible to wear the electronics unit 14 e.g. in a pocket or clothing.

Fig. 6 shows an architecture of the system 10 where a power supply 50 (e.g. a battery or accumulator pack) is arranged separately from the sensor unit 12 and from the electronics unit 14 and is provided with a connector cable 52 for energy transfer. Thus, replacement or recharging only of the power supply 50 is easily feasible. In this case, the electronics unit 14 may contain an energy buffer.

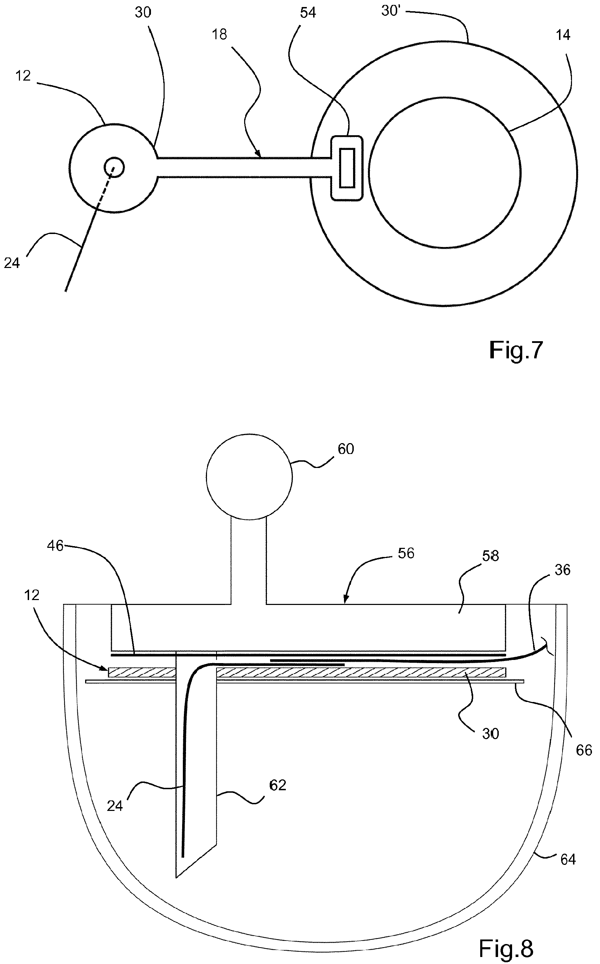

Fig. 7 shows an embodiment in which the connection means 18 is formed without a cable by a board-to-board connector 54 integrated to an extension of the plaster pad 30 which carries the sensor unit 12 and to the plaster pad 30' which carries the electronics unit 14. Here, the plaster includes thin film conductive elements. In the same way it is possible to integrate conductive paths and connector into a plaster that carries the power supply 50.

As shown in Fig. 8, an auxiliary component of the system 10 may comprise an inserter 56 for placing the sensor unit 12 at a selected site of the skin 32 while inserting the sensor into subcutaneous tissue. In a simple implementation, the inserter 56 comprises a stamp 58, a manual handle 60 and a slotted cannula 62 for receiving the distal portion of the sensor 24. The inserter may be provided with a sterile container 64 covering the cannula 62. Alternatively, a protecting cap may receive the cannula 62. A strippable liner 66 covers the adherent side of the pad 30 prior to application, while the covering foil 46 may be coated by a transfer paste which only lightly sticks to the stamp 58. Then, after insertion, only the entity comprising the stamp 58 and the cannula 62 is retracted, such that the sensor unit 12 remains on the skin.

As illustrated in Fig. 1, the receiver 20 may be provided in the form of a smartphone 66 with a monitoring software provided as an app 68 downloadable from the internet to embed the smartphone 66 into the medical system 10. For communication of the analyte data the wireless connection 22 may include a first type of transmitter unit, preferably a Bluetooth Low Energy (BLE) unit allowing for relatively long range continuous data communication (i.e. frequently or real time in regular or irregular intervals). Another option may comprise a second type of transmitter unit, preferably a Near Field Communication (NFC) unit, where data communication is triggered whenever the receiver is brought in proximity of the electronics unit to allow for data on demand by the user. These two types of transmitter units may be fully interchangeable giving the user the flexibility to choose which transmitter suits the best. Particularly, the second type of transmitter unit builds much smaller due to less power consumption and is therefore more discrete to wear.

## Claims

1. A medical system for monitoring an analyte in a body fluid comprising
a sensor unit (12) including a flexible pad (30) attachable to a body of a user and a sensor (24) which has a subcutaneously implantable electrode portion (26) and a contact portion (28) arranged on the pad (30),
an electronics unit (14) configured for receiving a measurement signal from the sensor unit (12),
and a power supply unit (50) for at least powering the electronics unit (14)
**characterized in that**
the sensor unit (12) and the electronics unit (14) are arranged as physically independent entities which in use are disposed at a distance from each other at different sites on or near the body,
wherein the sensor unit (12) is construed without rigid mounting base on the pad and a connection means (18) is provided between the sensor unit (12) and the electronics unit (14) at least for transferring the measurement signal.

2. The medical system of claim 1, wherein the connection means (18) is formed as a flexible connection cable (36).

3. The medical system of claim 2, wherein the connection cable (36) has a first plug-free end (38) which is non-detachably joined to the contact portion (28) of the sensor (24) to permanently maintain an electrical connection.

4. The medical system of claim 2 or 3, wherein a second end (40) of the connection cable (36) is provided with a plug connector (42) preferably formed as a flat quick-connect termination, and wherein the electronics unit (14) has a plug socket (44) to receive the plug connector (42).

5. The medical system according to any of claims 2 to 4, wherein the connection cable (36) has a length in the range of 3 to 30 cm, preferably 5 to 10 cm.

6. The medical system of claim 1, wherein the connection means (18) is formed without a cable by a board-to-board connector (54) including electric lines integrated to a plaster pad (30) which carries at least one of the sensor unit (12), the electronics unit (14) and the power supply unit (50).

7. The medical system according to any of claims 1 to 6, wherein the sensor unit (12) is formed as a flat layered assemblage which has a height of less than 3 mm, preferably less than 1 mm.

8. The medical system according to any of claims 1 to 7, wherein the sensor unit (12) has a lateral dimension of less than 5 cm, preferably less than 2 cm and most preferably less than 1 cm.

9. The medical system according to any of claims 1 to 8, further comprising a reusable or disposable inserter (56) for placing the sensor unit (12) on the skin while inserting the subcutaneously implantable electrode portion (26) of the sensor into subcutaneous tissue.

10. The medical system according to any of claims 1 to 9, wherein the power supply unit (50) is arranged separately from the sensor unit (12) and from the electronics unit (14) and is provided with a cable-based or wireless energy transfer means (52).

11. The medical system according to any of claims 1 to 10, wherein the electronics unit (14) comprises a transmitter for preferably wireless transmission of analyte data.

12. The medical system according to any of claims 1 to 11, further comprising a receiver (20) configured to receive analyte data from the electronics unit (14).

13. The medical system of claim 12, wherein the receiver (20) is provided as a customized remote control device or as a smartphone (66) including control software (68) in the form of a mobile app.

14. The medical system according to any of claims 1 to 13, wherein the electronics unit (14) and/or the power unit is attachable to the body via a hook-and-loop plaster connection (48).

15. The medical system according to any of claims 1 to 14, wherein the electronics unit (14) is a portable device which can be carried by the user for example in a pocket or in the form of a watch.
